# EUROPEAN PATENT SPECIFICATION

(11) **EP 4 456 019 B1**
(45) Date of publication and mention of the grant of the patent: **06.05.2026**
(21) Application number: 24171518.4
(22) Date of filing: 22.04.2024
(51) Int. Cl.: G06V 40/10, G06V 10/762, A43D 1/02

(54) **BODY SHAPE ANALYSIS DEVICE AND BODY SHAPE ANALYSIS METHOD**
KÖRPERFORMANALYSEVORRICHTUNG UND KÖRPERFORMANALYSEVERFAHREN
DISPOSITIF D'ANALYSE DE FORME CORPORELLE ET PROCÉDÉ D'ANALYSE DE FORME CORPORELLE

(30) Priority: 27.04.2023 JP 2023073079
(43) Date of publication of application: 30.10.2024
(73) Proprietor: ASICS Corporation, Kobe-shi, Hyogo 650-8555 (JP)
(72) Inventor: SAKAGUCHI, Masanori, Kobe-shi, 6508555 (JP); TAKASHIMA, Shingo, Kobe-shi, 6508555 (JP); HATANO, Genki, Kobe-shi, 6508555 (JP)
(74) Representative: TBK

(56) References cited:
- LEE YU-CHI ET AL: "Taiwanese adult foot shape classification using 3D scanning data", vol. 58, no. 3, 31 October 2014 (2014-10-31), GB, pages 513 - 523, XP093189864, ISSN: 0014-0139, Retrieved from the Internet <URL:https://www.tandfonline.com/doi/pdf/10.1080/00140139.2014.974683> [retrieved on 20240725], DOI: 10.1080/00140139.2014.974683

## Description

### BACKGROUND

### Technical field

The present disclosure relates to a body shape analysis device and a body shape analysis method.

### Background Information

It is preferable to prepare many types of shoe size variations for various foot shapes and sizes. However, since increasing the size variations entails an increase in manufacturing costs and the risk of holding a large stock, many shoe manufacturers mainly prepare size variations that are in high demand.

With regard to the shoe size description, since the unit and standards of size vary depending on the country or region, when shoes of the same size are sold in different countries or regions, the size description needs to be adjusted for each country or region. Accordingly, it is difficult to strictly conform the size description of shoes sold internationally to the size description in every country or region. Therefore, it may be sometimes difficult to know which size fits your feet without actually trying the shoes on. Some shoe stores may be staffed with a professional called a shoe fitter who can select shoes that fit a customer's feet, based on a wealth of knowledge and experience. However, online sales cannot rely on shoe fitters. Meanwhile, in recent years, there has been developed a technology of measuring foot shapes three-dimensionally to select shoes that fit the feet.

There is a known technology of selecting a type of shoe that fits a foot shape, based on three-dimensional data obtained by measuring the foot shape three-dimensionally (see Japanese Unexamined Patent Application Publication JP 2000-90272 A, for example). In Patent Literature 1, from the three-dimensional foot shape data, feature data of a two-dimensional shape is generated for each of the toe portion, central portion, and heel portion. Similarly, feature data for each portion is also generated for lasts. The feature data of a large number of lasts are collected and clustered, and each cluster is stored with a code assigned thereto. The relationship between the feature data of a foot shape and the feature data of a last that fits the foot shape is learned with a neural network, and a rule is defined such that the feature data of a last is obtained from the feature data of a foot shape.

Furthermore, the scientific article "Taiwanese adult foot shape classification using 3D scanning data" by Lee Yu-Chi et al, in "ERGONOMICS" vol. 58 no. 3, pages 213-523, refers to classifying shoe shapes of Taiwanese adults by performing 3D foot scanning of 3000 persons. Thereby, eight CCD cameras and four laser projectors are used for constructing a 3D foot model. Then, the obtained data is used for statistical analysis and classifying the foot shapes.

In general, commercially available shoes are often made separately for men and women, taking into consideration differences in physical size and design preferences between men and women. In Japan, in particular, size variations are often provided separately for men and women in accordance with the Japanese Industrial Standards (JIS), so that shoes are rarely designed as unisex models. Except for sports shoes for which the fit is particularly important, size variations based on the foot width, besides the foot length, are rarely prepared.

When shoes are selected based on the foot length, a mismatch may occur such that the foot width is too narrow or too wide. Also, there may be a case where, because of lack of fit in a part other than the foot length or foot width, shoes that fit the feet cannot be found. However, since size variations are basically provided based on the foot length, which is the factor having the greatest impact on the fit of shoes, it would be complicated to increase the shoes to be considered for purchase by adding shoes of different foot length sizes, even though the fit in the foot width or other parts is considered important. Especially, in online sales, trying multiple sizes is not easy.

Meanwhile, for shoe manufacturers, since there are differences in demand for sizes and shapes due to differences in average physical size among countries or regions, it is particularly difficult to prepare size variations that meet the demand for shoes to be sold internationally.

### SUMMARY

The present disclosure has been made in view of such a situation, and it is an object thereof to provide a technology for improving the fit between a foot and a shoe.

According to the invention this object is achieved by a body shape analysis device according to claim 1 and a body shape analysis device according to claim 5. Further features and advantageous modifications are shown in the dependent claims.

In response to the above issue, a body shape analysis device according to one embodiment of the present disclosure includes: a model storage unit that stores, in advance, a plurality of three-dimensional homologous models for a plurality of body shapes with a three-dimensional coordinate group indicating an anatomical feature of a foot, wherein a foot shape three-dimensional homologous model is a model with which multiple foot shapes having various sizes and shapes are each represented by a polyhedron anatomically defined by the same topological structure with the same number of points; a measurement data acquirer that acquires, as measurement data, a three-dimensional coordinate group indicating an anatomical feature corresponding to the three-dimensional homologous model of a body shape of a measurement subject; and an evaluation determination unit that determines, among the plurality of three-dimensional homologous models, a three-dimensional homologous model similar to a body shape of the measurement subject by evaluating similarity in coordinates between a three-dimensional coordinate group indicating an anatomical feature in the measurement data and a three-dimensional coordinate group indicating an anatomical feature in each of the plurality of three-dimensional homologous models. The plurality of three-dimensional homologous models are classified into a plurality of clusters by cluster analysis based on cosine similarity between position vectors from a predetermined reference point to the respective points in the three-dimensional homologous models and are stored in the model storage unit.

Yet another embodiment of the present disclosure relates to a body shape analysis method. The method includes: reading a plurality of three-dimensional homologous models for a plurality of body shapes with a three-dimensional coordinate group indicating an anatomical feature of a foot, wherein a foot shape three-dimensional homologous model is a model with which multiple foot shapes having various sizes and shapes are each represented by a polyhedron anatomically defined by the same topological structure with the same number of points; acquiring, as measurement data, a three-dimensional coordinate group indicating an anatomical feature corresponding to the three-dimensional homologous model of a body shape of a measurement subject; evaluating similarity in coordinates between a three-dimensional coordinate group indicating an anatomical feature in the measurement data and a three-dimensional coordinate group indicating an anatomical feature in each of the plurality of three-dimensional homologous models; and determining, among the plurality of three-dimensional homologous models, a three-dimensional homologous model similar to a body shape of the measurement subject, based on evaluation for the similarity. The plurality of three-dimensional homologous models are classified into a plurality of clusters by cluster analysis based on cosine similarity between position vectors from a predetermined reference point to the respective points in the three-dimensional homologous models and are stored in the model storage unit.

### BRIEF DESCRIPTION OF THE DRAWINGS

Embodiments will now be described, by way of example only, with reference to the accompanying drawings which are meant to be exemplary, not limiting, and wherein like elements are numbered alike in several Figures, in which:
Fig. 1 illustrates a basic configuration of a shape analysis system;
Fig. 2 is a functional block diagram that shows each configuration in a shape analysis device;
Fig. 3 shows an example of a three-dimensional homologous model;
Figs. 4A and 4B each show the relationship between the distribution of foot lengths and ball girths of women or men and the JIS in Japan;
Fig. 5 shows a difference between a general cluster analysis method and a new cluster analysis method;
Fig. 6 shows a method in which different cluster analysis methods for an upper layer and a lower layer are combined;
Fig. 7 shows the Euclidean distance and cosine similarity between points;
Figs. 8A and 8B show the relationships between corresponding points in two foot shape models;
Figs. 9A and 9B each show comparison between foot shape models using the Euclidean distance and the cosine similarity;
Fig. 10 shows differences in classification result between cluster analysis based on the cosine similarity and cluster analysis based on the Euclidean distance;
Fig. 11 shows a classification result of the cluster analysis based on the cosine similarity;
Fig. 12 shows regression lines of the foot length and ball girth of top three clusters and also shows results of principal component analysis of the top three clusters;
Fig. 13 shows results of principal component analysis for a cluster;
Fig. 14 illustrates a basic configuration of a shape analysis system; and
Fig. 15 is a functional block diagram that shows each configuration in a shape analysis device.

### DETAILED DESCRIPTION

The invention will now be described by reference to the preferred embodiments. This does not intend to limit the scope of the present invention, but to exemplify the invention.

In the following, the present disclosure will be described based on preferred embodiments with reference to each drawing. In the embodiments and modifications, like reference characters denote like or corresponding constituting elements, and the repetitive description will be omitted as appropriate.

### First Embodiment

In the present embodiment, three-dimensional shapes of feet of a large number of measurement subjects are measured and accumulated as foot shape data, which are then classified into multiple clusters using a cluster analysis method described later. Also, by designing lasts or sock liners based on the classified multiple clusters, shape variations of shoe products can be provided based on standards different from conventional standards, and shoes that fit many people's feet can be provided. Further, demand for each shape in the shape variations is forecasted based on a foot shape distribution.

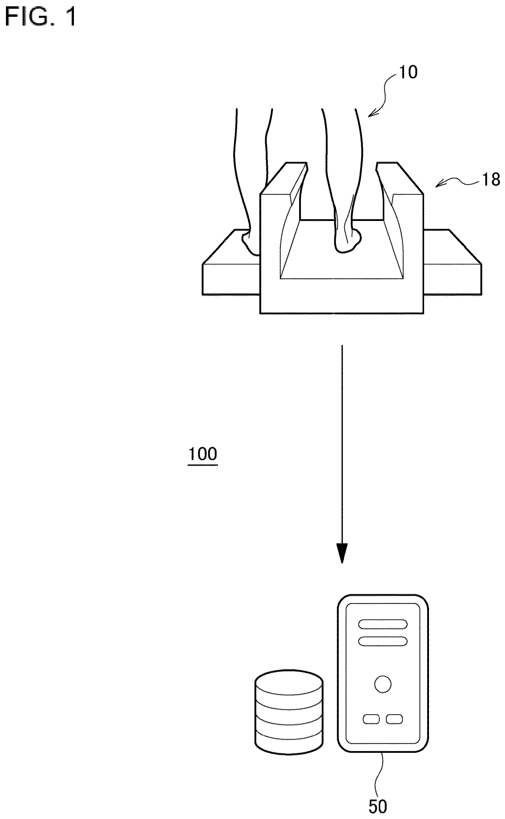

Fig. 1 illustrates a basic configuration of a shape analysis system 100. The shape analysis system 100 includes a three-dimensional foot shape measuring device 18 and a shape analysis device 50. A measurement subject 10 scans his or her foot shape using the three-dimensional foot shape measuring device 18. For opportunities to acquire foot shape data, for example, a large number of subjects may be selected as measurement subjects 10 at the shoe design stage, for the purpose of acquiring foot shape data of various sizes or shapes regardless of gender or age, or a person who is considering purchase of shoes at a shoe store may be selected as a measurement subject 10.

The three-dimensional foot shape measuring device 18 acquires three-dimensional data related to a foot shape of the measurement subject 10 by laser measurement. The "foot shape" as used herein is a three-dimensional model that virtually reproduces a three-dimensional shape of a foot of the measurement subject 10. The measurement values as the result of three-dimensional measurement of a foot shape scanned by the three-dimensional foot shape measuring device 18 are transmitted from the three-dimensional foot shape measuring device 18 to the shape analysis device 50. The shape analysis device 50 may be implemented as a server to which multiple three-dimensional foot shape measuring devices 18 connect via a network and that collects and analyzes measurement data from each of the multiple three-dimensional foot shape measuring devices 18. The "body shape analysis device" in the claims may mean the entire shape analysis system 100 or may mean the shape analysis device 50. Also, in the present embodiment, the shape analysis device 50 substantially corresponds to the "body shape analysis device" because many of the characteristic functions included in the "body shape analysis device" in the claims are implemented such as to be included in the shape analysis device 50.

The shape analysis device 50 is a server computer connected to multiple three-dimensional foot shape measuring devices 18 via a network line, such as the Internet or a local area network (LAN), and a communication means, such as wireless communication. The shape analysis device 50 may be constituted by a single server computer or may be constituted by a combination of multiple server computers.

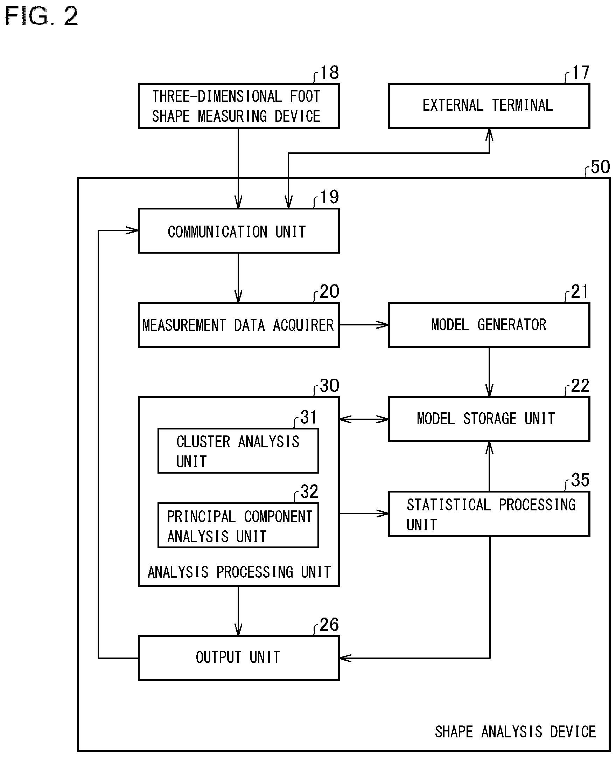

Fig. 2 is a functional block diagram that shows each configuration in the shape analysis device 50. The shape analysis device 50 may be implemented by various hardware configurations and software configurations. With regard to the shape analysis device 50, Fig. 2 shows functional blocks implemented by coordination of various hardware configurations and software configurations. Therefore, it will be understood by those skilled in the art that these functional blocks may be implemented in a variety of forms by hardware only, software only, or a combination thereof. The shape analysis device 50 is constituted by a combination of hardware, such as a microprocessor, memory, display, and communication module. The shape analysis device 50 may be a server computer constructed and managed by a shoe manufacturer. On the shape analysis device 50, a program having the following functions runs. The shape analysis device 50 includes, as its functions, a communication unit 19, a measurement data acquirer 20, a model generator 21, a model storage unit 22, an output unit 26, an analysis processing unit 30, and a statistical processing unit 35. The communication unit 19 of the shape analysis device 50 is connected to the three-dimensional foot shape measuring device 18 and an external terminal 17 via a network.

The model storage unit 22 stores, in advance, multiple three-dimensional models for multiple body shapes with a three-dimensional coordinate group indicating anatomical features of at least part of a body. The body shape as used herein mainly means a foot shape but may also mean a body shape other than a foot shape, such as physical type data indicating the shape of the entire human body, or a partial body shape other than a foot shape. The three-dimensional model of a foot shape stored in the model storage unit 22 is, for example, a three-dimensional homologous model in which a coordinate group of points indicating anatomical features of an average foot in three-dimensional space is defined. A foot shape three-dimensional homologous model is a model with which multiple foot shapes having various sizes and shapes are each represented by a polyhedron anatomically defined by the same topological structure with the same number of points. The points representing anatomical features of an average foot are obtained in advance based on foot samples obtained from a large number of subjects. The three-dimensional models in modifications may be various other models created in accordance with the same anatomical criteria, such as a three-dimensional shape model in a spherical coordinate system, and a model obtained by modifying a template foot shape generated by a three-dimensional CAD system, based on anatomical landmarks.

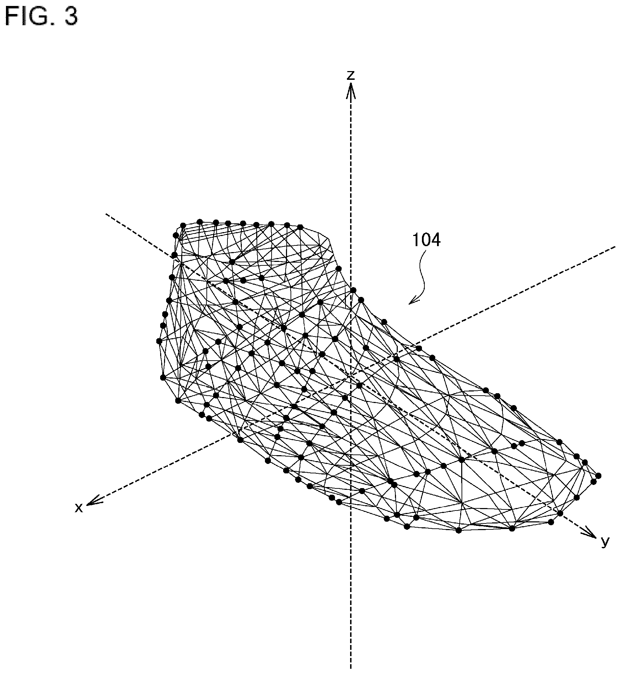

Fig. 3 shows an example of the three-dimensional homologous model. A three-dimensional homologous model 104 of a foot shape is constituted by a point cloud representing anatomical features of a foot. The three-dimensional homologous model 104 does not necessarily faithfully reproduce the foot shape, especially the shape near the toes, of the measurement subject 10 and is intentionally deformed so as to be easily compared with other foot shapes or three-dimensional homologous models. More specifically, the three-dimensional homologous model 104 does not have complete similarity to the actual foot shape or foot contour of the measurement subject 10 and has a shape such that convex hull processing has been performed particularly on the contour of the toes. Also, by detecting multiple characteristic points included in the foot contour of the three-dimensional homologous model 104 and using each point as a reference, the length of each part, such as the foot length, ball girth, instep girth, short heel girth, and heel width, can be measured. Since the methods for measuring the length of each part is known, the description thereof will be omitted.

Referring back to Fig. 2, the measurement data acquirer 20 acquires, as measurement data, a three-dimensional coordinate group indicating anatomical features corresponding to a three-dimensional homologous model of the foot shape of the measurement subject 10, from the three-dimensional foot shape measuring device 18 via the communication unit 19. The measurement data acquirer 20 may acquire measurement data from an external terminal 17 other than the three-dimensional foot shape measuring device 18 via the communication unit 19. The model generator 21 generates a foot shape three-dimensional homologous model based on the measurement data acquired by the measurement data acquirer 20 and stores the model as a new three-dimensional homologous model in the model storage unit 22. In this case, even when measurement data is acquired using various types of foot shape measurement devices as the three-dimensional foot shape measuring device 18 or external terminal 17, the measurement data is converted into a three-dimensional homologous model by the model generator 21. This can generalize the measurement data into a data format that can be easily used. Meanwhile, in a modification, the measurement data to be acquired by the measurement data acquirer 20 may be made into a foot shape three-dimensional homologous model format in advance by the three-dimensional foot shape measuring device 18 or external terminal 17. In such a case, the model generator 21 need not generate a foot shape three-dimensional homologous model based on the measurement data, and the measurement data may be stored as it is as a new three-dimensional homologous model in the model storage unit 22.

The analysis processing unit 30 includes a cluster analysis unit 31 and a principal component analysis unit 32. The cluster analysis unit 31 performs cluster analysis for an upper layer and a lower layer, which will be described later, on multiple three-dimensional homologous models stored in the model storage unit 22. The cluster analysis unit 31 performs a method in which different cluster analysis methods for the upper layer and lower layer are combined. The principal component analysis unit 32 performs principal component analysis, which will be described later, on multiple foot shape three-dimensional homologous models stored in the model storage unit 22. After performing the cluster analysis for the upper layer, the cluster analysis unit 31 performs the cluster analysis for the lower layer based on the result of the principal component analysis performed by the principal component analysis unit 32.

As the cluster analysis unit 31 performs the cluster analysis and the principal component analysis unit 32 performs the principal component analysis, information of each cluster is acquired as the classification results and output to the outside by the output unit 26. Based on the classification results of the cluster analysis, the analysis processing unit 30 divides the three-dimensional homologous models stored in the model storage unit 22 into multiple clusters.

The output unit 26 may display the classification results on a display screen of the shape analysis device 50 or an external terminal 17 or may transmit the classification results to an external terminal 17 via the communication unit 19. The output unit 26 outputs three-dimensional coordinate groups of three-dimensional homologous models of each cluster. The information of the classification results output by the output unit 26 may be used for designing of lasts or sock liners.

The statistical processing unit 35 performs demand forecasting for a last corresponding to each foot shape model based on the foot shape model distribution for each cluster. The result of the demand forecasting is output to the external terminal 17 by the output unit 26. Based on the result of the demand forecasting, the statistical processing unit 35 manages a database of lasts, footwear, or sock liners corresponding to the foot shape models in each cluster stored in the model storage unit 22.

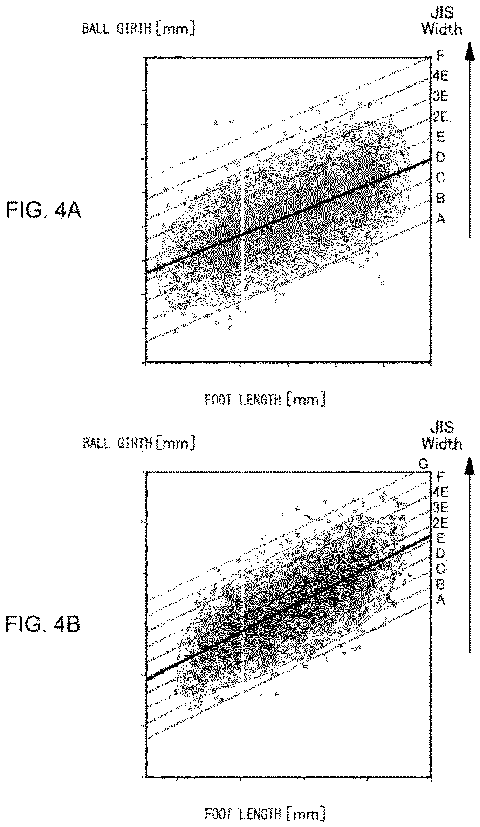

Figs. 4A and 4B each show the relationship between the distribution of foot lengths and ball girths of women or men and the JIS in Japan. Shoe sizes in Japan are classified based on the foot length and the ball girth/foot width and standardized according to the Japanese Industrial Standards (JIS) as a standard specification. In this standard, sizes are defined separately for men, women, and children, taking into consideration the differences in physical size.

Fig. 4A shows the JIS for women and a distribution obtained from samples of a large number of adult women. Fig. 4B shows the JIS for men and a distribution obtained from samples of a large number of adult men. The JIS specifies the foot length in the range of 19.5 cm to 27.0 cm for women, whereas it specifies the foot length in the range of 20.0 cm to 30.0 cm for men. For the ball girth/foot width, shoe widths A-F for women and shoe widths A-G for men are defined for each foot length. However, the ranges are defined differently, such as the shoe width A for women is defined as 183 mm in ball girth and 76 mm in foot width, whereas the shoe width A for the foot length of 20.0 cm for men is defined as 189 mm in ball girth and 79 mm in foot width.

The multiple diagonal lines ascending to the right in Figs. 4A and 4B respectively indicate the relationships between the foot length [mm] and ball girth [mm] defined for the respective foot length sizes in the JIS. The multiple diagonal lines in Fig. 4A respectively indicate the shoe widths A-F for women from the bottom, and the multiple diagonal lines in Fig. 4B respectively indicate the shoe widths A-G for men from the bottom. The difference in ball girth between adjacent shoe widths is about 6 mm.

The distribution of the women's samples shown in Fig. 4A mostly lies within the range of the foot length and ball girth specified in the JIS, but there are some samples outside the range. Also, the distribution of the men's samples shown in Fig. 4B mostly lies within the range of the foot length and ball girth specified in the JIS, but there are some samples outside the range, as in the case of women's samples.

The bold diagonal line in each of Figs. 4A and 4B indicates a sample average for women or men. There is variance of ball girth of about ± 3-4 widths each above and below the average line for each foot length size. That is, even if the relationship between the foot length and ball girth is defined and provided as a standard, as shown in each of Figs. 4A and 4B, the variance or dispersion of the ball girth is actually large. Moreover, although the conventional shoe standards only define the relationships between the foot length and ball girth, the size and shape of a foot are determined further based on other parameters. Accordingly, even if only the foot length and the ball girth fit, the target shoes may not necessarily fit the subject's feet. In order to solve such a situation, a method described below is used to classify a group of foot shape models, measured three-dimensionally, by cluster analysis and to design size variations based on the classification.

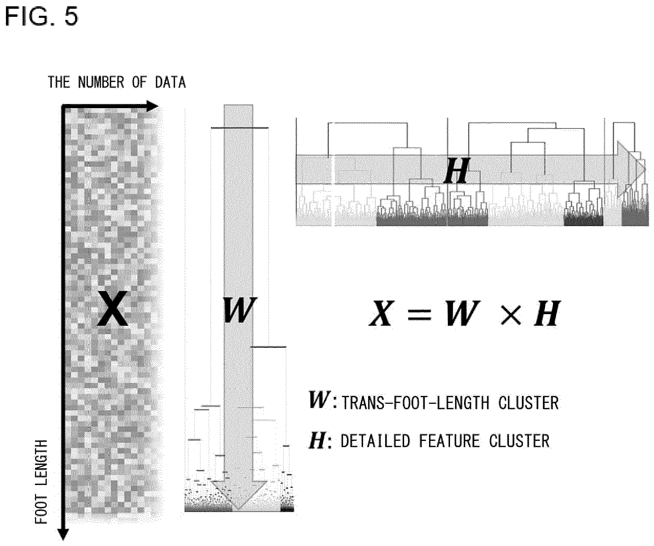

Fig. 5 shows a difference between a general cluster analysis method and a new cluster analysis method. When a general cluster analysis method is used for clustering of a foot shape data group X, it is conceivable to perform cluster analysis based on the Euclidean distance. However, with this method, cluster classification is performed in a way that is greatly affected by the foot length size. Also, when the classification needs to be performed based on not only the foot length size but also differences in foot shape, it is conceivable to divide the data based on the foot length size and then perform cluster analysis for each foot length size. However, even with this method, it is expected that classification will be greatly affected by the foot length size and it will be difficult to make a difference from a conventional grading method in which classification is performed only based on multiple ball girth sizes for each foot length size. In addition, the number of classified clusters may vary depending on the size.

Therefore, the cluster analysis unit 31 in the present embodiment performs cluster analysis across all foot length sizes, as shown in Fig. 5. The foot shape data group X is configured such that a large cluster W, obtained by classification based on similarity in shape (i.e., shapes like similar figures) across foot length sizes, is multiplied by a small cluster H, obtained by classification based on detailed features. The next figure illustrates a hierarchical state of such clusters.

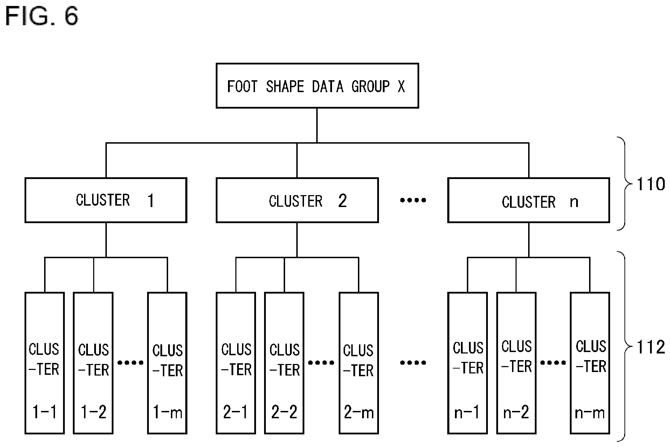

Fig. 6 shows a method in which different cluster analysis methods for an upper layer and a lower layer are combined. First, the foot shape data group X is classified, by cluster analysis based on the cosine similarity, into clusters 1 to n, which constitute a first layer 110 as the upper layer. The clusters 1 to n are each subdivided, by cluster analysis based on principal component analysis in which the results are dimensionally reduced to the second and the subsequent principal components excluding the first principal component, into clusters (1-1, 1-2, ..., 1-m, 2-1, 2-2, ..., 2-m, ..., n-1, n-2, ..., n-m), which constitute a second layer 112 as the lower layer. The cluster analysis method for each of the upper layer and lower layer will be described later.

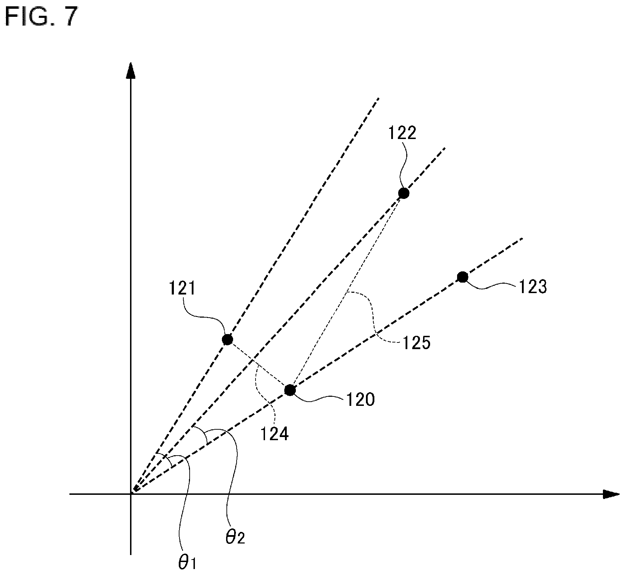

Fig. 7 shows the Euclidean distance and the cosine similarity between points. Explanation will be given using an example in which there are four points of a first point 120, a second point 121, a third point 122, and a fourth point 123 in a vector space. A common method for measuring the proximity between two points is a method using distance called the Euclidean distance for judgment. The Euclidean distance is the linear distance between two points, and a smaller value thereof means that the two points are more similar. Meanwhile, the present embodiment mainly employs a method using similarity called the cosine similarity for judgment. The cosine similarity is the cosine value (cosθ) of an angle θ formed by position vectors of two points from the origin, and, when the angle θ is smaller, the cosine value becomes closer to 1, which means that the two points are more similar. In general, the cosine similarity is used in situations where the similarity between sentences is measured and is rarely used in situations where the similarity between shapes is measured.

The Euclidean distance (distance 124) from the first point 120 to the second point 121 is shorter than the Euclidean distance (distance 125) from the first point 120 to the third point 122. Therefore, the second point 121 is judged closer to the first point 120 than the third point 122 based on the Euclidean distance.

On the other hand, the angle θ2 between the position vectors of the first point 120 and the third point 122 from the origin is smaller than the angle θ1 between the position vectors of the first point 120 and the second point 121 from the origin, and the cosine value of the angle θ2 is closer to 1. Therefore, the third point 122 is judged closer to the first point 120 than the second point 121 based on the cosine similarity.

Further, the first point 120 and the fourth point 123 have almost the same position vectors from the origin and hence the angle θ is almost zero, so that the cosine similarity is almost 1. Therefore, the fourth point 123 is farther from the first point 120 than the second point 121 based on the Euclidean distance but is infinitely closer to the first point 120 based on the cosine similarity.

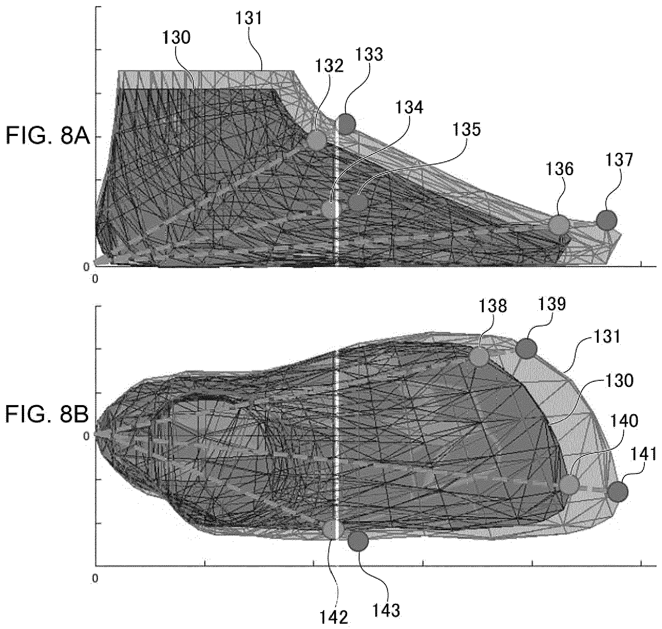

Figs. 8A and 8B show the relationships between corresponding points in two foot shape models. The foot shape models are constituted by point clouds, and each point represents an anatomical feature in the foot shape. Since each foot shape model includes the same points of anatomical features, the similarity between the overall foot shape models is determined based on the similarity of the positional relationships between the points. When a relatively small foot shape model 130 and a relatively large foot shape model 131 are compared, the cosine similarity is calculated based on the angle between the position vectors, from the origin, of corresponding points in the clouds of a large number of points included in the respective foot shape models. In the side view of Fig. 8A, points 132 and 133, points 134 and 135, and points 136 and 137 correspond to each other; in the top view of Fig. 8B, points 138 and 139, points 140 and 141, and points 142 and 143 correspond to each other. In Fig. 8A, each of the angle between the position vectors of the points 132 and 133, the angle between the position vectors of the points 134 and 135, and the angle between the position vectors of the points 136 and 137 is almost zero. In Fig. 8B, each of the angle between the position vectors of the points 138 and 139, the angle between the position vectors of the points 140 and 141, and the angle between the position vectors of the points 142 and 143 is almost zero. Therefore, although they differ in size, the foot shape model 130 and the foot shape model 131 can be said to be almost similar figures.

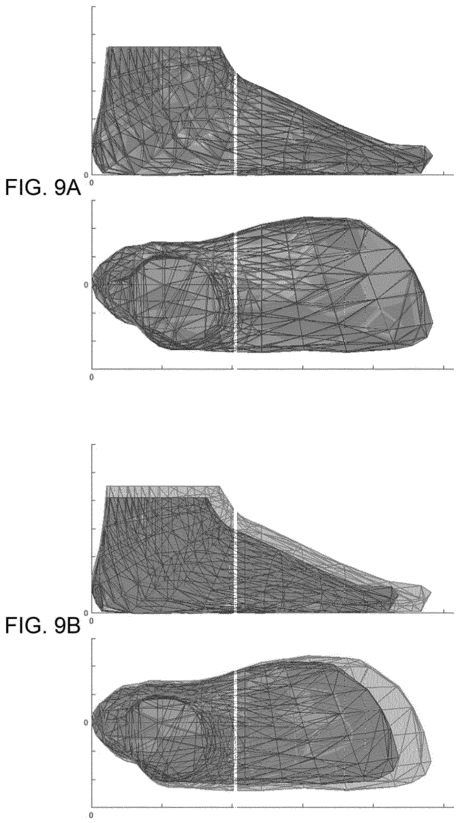

Figs. 9A and 9B each show comparison between foot shape models using the Euclidean distance and the cosine similarity. When foot shape models are closer in foot length size, the value of the Euclidean distance becomes smaller, so that the models are likely to be judged "similar". Conversely, foot shape models with a larger value of the Euclidean distance are likely to be judged "dissimilar". When foot shape models are closer to similar figures, the cosine similarity becomes closer to 1, so that the models are likely to be judged "similar". Conversely, when the cosine similarity is closer to 0, the models are likely to be judged "dissimilar".

In the example of Fig. 9A, the two foot shape models almost overlap. The Euclidean distance is relatively small, and the cosine similarity is almost 1. Therefore, based on either the Euclidean distance or the cosine similarity, the models are likely to be judged "similar".

In the example of Fig. 9B, on the other hand, the two foot shape models are clearly different in size. Since the Euclidean distance is relatively large, the models are likely to be judged "dissimilar" based on the Euclidean distance. Meanwhile, since the cosine similarity is almost 1, which is almost the same as in the example of Fig. 9A, the models are likely to be judged "similar" based on the cosine similarity. That is, when two foot shape models are compared, even when there is a large difference based on the Euclidean distance, there may be almost no difference based on the cosine similarity, indicating that the cosine similarity is not affected by size. The example of Fig. 9B shows that the two foot shape models are different in size but are similar such that they can be said to be almost similar figures.

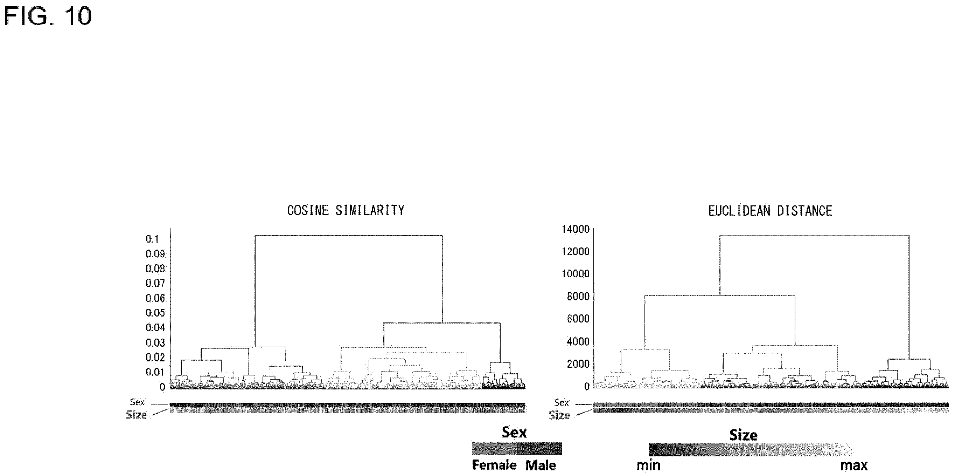

Fig. 10 shows differences in classification result between the cluster analysis based on the cosine similarity and the cluster analysis based on the Euclidean distance. As the classification results of actual cluster analysis of the foot shape data group, hierarchical clusters based on the Euclidean distance are shown on the right, and the hierarchical clusters based on the cosine similarity are shown on the left.

In the cluster analysis based on the Euclidean distance, the foot length gradually increases from left to right in the subdivided clusters in the lowest layer, with the foot length size being smaller toward the left and being larger toward the right. This indicates that the influence of the foot length size is dominant in the cluster analysis based on the Euclidean distance. Also, in terms of sex, males gradually increases from left to right, with females increasing toward the left and males increasing toward the right. This is because women tend to have smaller foot lengths than men, and it can be seen, as a result, that the cluster analysis based on the Euclidean distance is also affected by sex.

Meanwhile, in the cluster analysis based on the cosine similarity, various foot lengths are randomly mixed from left to right in the subdivided clusters in the lowest layer, indicating that there is little influence by the foot length size. Similarly, sex is also randomly mixed, indicating that there is also little influence by sex.

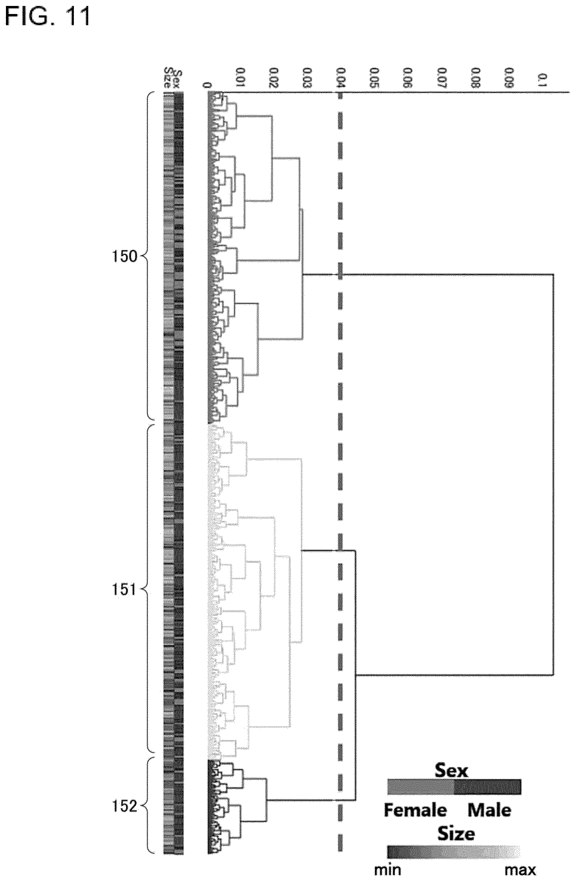

Fig. 11 shows a classification result of the cluster analysis based on the cosine similarity. The samples for the cluster analysis were foot shape data of about 5,000 adult males and females. A hierarchical clustering method using the Ward's method based on the cosine similarity between points included in foot shape data was adopted.

The top three clusters (a first cluster 150, a second cluster 151, and a third cluster 152) are regarded as the upper layer. In each of the first cluster 150, second cluster 151, and third cluster 152, a foot shape based on the coordinates of each point obtained by normalizing the foot length size is defined as an average foot.

By superimposing the foot shape model of the average foot of each cluster, the characteristics of each cluster and the areas where such characteristics are likely to appear can be determined based on differences in partial shapes. For example, a characteristic may be found in a difference in instep height, such as, in a direction of instep height, the instep of the first cluster 150 is the lowest, the instep of the second cluster 151 is intermediate, and the instep of the third cluster 152 is the highest. For example, a characteristic may be found in a difference in first toe angle, such as, with regard to the angle of the first toe, the angle of the first cluster 150 extends toward the medial side the most, the angle of the second cluster 151 is intermediate, and the angle of the third cluster 152 is slightly inclined toward the second toe. For example, a characteristic may be found in the inclination around the ankle, such as, with regard to the inclination around the ankle, the inclination of the first cluster 150 is the largest on the medial side, the inclination of the second cluster 151 is intermediate, and the inclination of the third cluster 152 is the smallest.

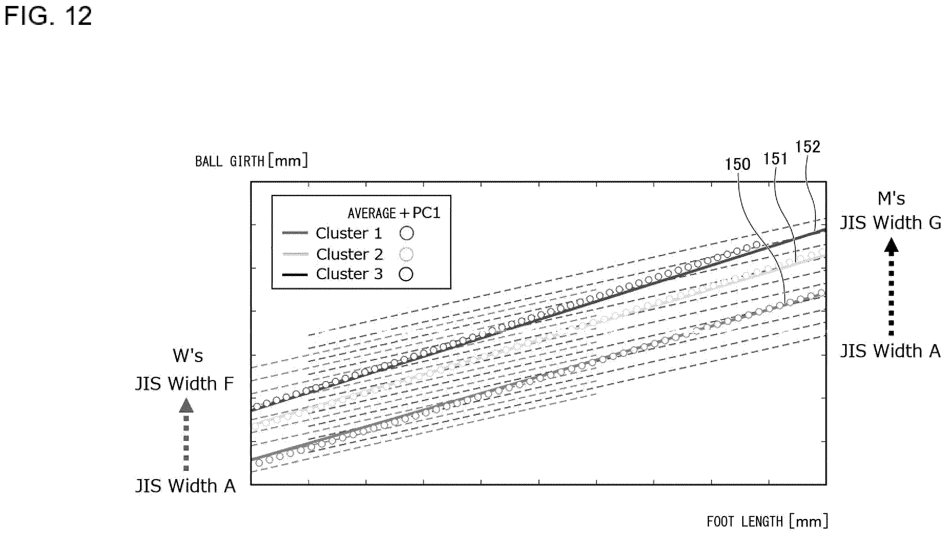

Fig. 12 shows regression lines of the foot length and ball girth of top three clusters and also shows results of principal component analysis of the top three clusters. Fig. 12 shows the regression lines that respectively show the relationships between the foot length and the ball girth in the first cluster 150, second cluster 151, and third cluster 152, superimposed on graphs of the JIS similar to those in Fig. 4. It can be seen therein that although the regression lines are almost parallel to each other, the slopes thereof are significantly different from the slopes of the regression lines showing the relationships between the foot length and the ball girth in the JIS. Therefore, by designing last variations along the regression lines of the respective clusters, it is possible to design variations that are different from the conventional grading provided separately for men and women according to the JIS, i.e., variations separated based on foot shape characteristics other than the foot length size regardless of gender.

The principal component analysis unit 32 performs principal component analysis on the foot shape data in each cluster, calculates data obtained by adding a first principal component PC1, as the variance, to the average foot that is the center of gravity of each cluster, and plots the relationship between the foot length and the ball girth in the data on the graph of Fig. 12. The clusters have been obtained by primary clustering based on the cosine similarity and classified such that the influence of the foot length size can be ignored, but the data itself contains foot length information. Therefore, when the principal component analysis unit 32 performs the principal component analysis, a principal component greatly affected by the foot length is extracted as the first principal component PC1. However, since the data have been classified based on the cosine similarity into clusters having different average foot shapes as the large classifications in the upper layer, the classification based on principal components in the clustering for each cluster in the lower layer can be performed easily. In this regard, the plots with open circles in the graph, which indicate the relationship between the foot length and the ball girth in the data of the average foot + the first principal component PC1 for each cluster, are located almost along the regression line of each cluster, indicating that the results of the cluster analysis and the results of the principal component analysis are consistent.

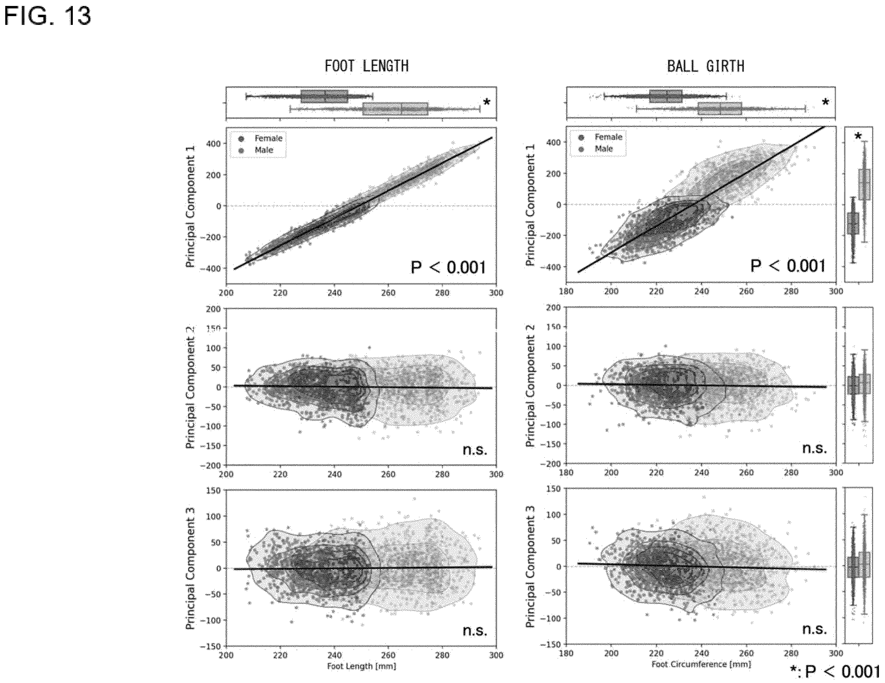

Fig. 13 shows results of principal component analysis for a cluster. When the principal component analysis unit 32 performs principal component analysis on a cluster, it can be seen what parameter has a significant influence on the variance of the data included in the cluster. In Fig. 13, the first principal component PC1 is set on the vertical axis in the upper graphs, a second principal component PC2 is set on the vertical axis in the middle graphs, and a third principal component PC3 is set on the vertical axis in the lower graphs. Also, the foot length is set on the horizontal axis in the left graphs, and the ball girth is set on the horizontal axis in the right graphs. In each graph, the plot group enclosed by a line on the left side indicates females, and the plot group enclosed by a line on the right side indicates males.

As shown in the two upper graphs on the left and right sides, the first principal component PC1 is strongly correlated not only with the foot length but also with the ball girth. On the other hand, as shown in the middle and lower graphs, the regression lines therein are almost horizontal, so that it can be seen that the second principal component PC2 and the subsequent principal component are hardly affected by the foot length or ball girth. Utilizing such characteristics in the results of the principal component analysis, in the present embodiment, the first principal component PC1, which is affected by the foot length and ball girth, is excluded, and only the second principal component PC2 and the subsequent principal components, which are not affected by the foot length or ball girth, are used for sub-clustering. The utilization of such characteristics is considered to be convenient for construction of variations based on the clustering focusing on differences in shape of a detail, which are different from the conventional grading based on the size.

As shown in Fig. 6, the first layer 110 as the upper layer includes multiple clusters classified by cluster analysis performed by the cluster analysis unit 31 based on the cosine similarity. Each of the clusters classified in the first layer 110 is further classified into many more detailed clusters in the second layer 112 as the lower layer, by cluster analysis performed by the cluster analysis unit 31 based on the principal component analysis with the dimensional reduction to the second and the subsequent principal components. In the principal component analysis in that case, the results of the principal component analysis performed by the principal component analysis unit 32 are dimensionally reduced to the second and the subsequent principal components excluding the first principal component. In the cluster analysis for the second layer 112, hierarchical clustering with the Ward's method based on the Euclidean distance is used based on the second principal component PC2 and the subsequent principal components of which the cumulative contribution ratio is up to 99%.

A group of foot shape models classified into a large number of clusters based on detailed features can be used not only for designing of last and footwear variations, but also for designing of sock liners. For example, variations associated with the shape of a detail, which does not depend on the foot length and ball girth sizes, may be absorbed by design variations of sock liners, thereby reducing the number of variations to be absorbed in the designing of lasts and footwear. That is, by combining the last and footwear variations and the sock liner variations, the overall variations may be designed to cover the entire foot shape model group.

Also, a large number of foot shape models included in the foot shape model group may be allocated to size variations according to the JIS, based on which of the lasts of size variations designed according to the JIS the foot shape model fits.

Since the number of people of the foot shape data included in each cluster is different, the ratio of the number of people in each cluster to the entire foot shape data group, as the population, is also different. Meanwhile, when a group of foot shape data collected and accumulated for various purposes are held as a database besides the foot shape data group used for the cluster analysis in the first embodiment, the distribution for each foot length size with respect to the population can be calculated based on those data. Therefore, the statistical processing unit 35 can quantify the ratio of each combination of a cluster and a foot length by combining the ratio of the number of people in each cluster and the distribution for each foot length size. The ratio of each combination of a cluster and a foot length when the foot length sizes are divided in the range of 22 cm to 31 cm in increments of 1 cm is quantified.

When a variation of a combination pattern for the size and shape of a footwear product or sock liner product is designed based on the ratio of each combination of a cluster and a foot length, the statistical processing unit 35 can forecast demand for initial stock according to the ratio of each combination of a cluster and a foot length. In such a case, a combination pattern of a cluster and a foot length for which demand is expected to be extremely low can be excluded from the shoe product variations from the beginning. Besides forecasting demand for initial stock, the statistical processing unit 35 can also reflect a demand forecast in the production plan when the stock decreases due to actual sales. The statistical processing unit 35 may output information regarding the ratio of each combination of a cluster and a foot length, to the outside via the output unit 26.

### Second Embodiment

The present embodiment differs from the first embodiment in that a three-dimensional shape of a foot of a measurement subject is measured to acquire foot shape data, and a foot shape similar thereto can be extracted from foot shape three-dimensional homologous models accumulated in the shape analysis device 50 and can be used as reference information for purchase of shoes. In the following, description will be given mainly for the differences from the first embodiment, and the explanation of features in common will be omitted.

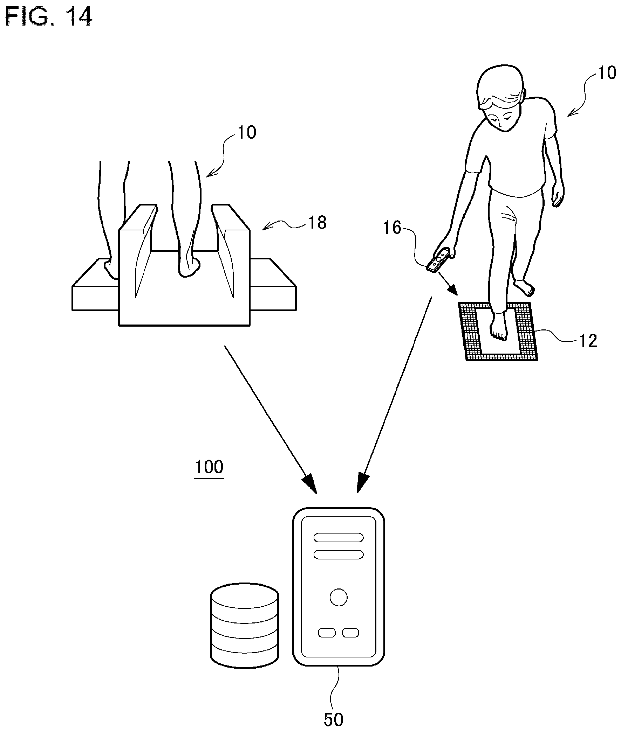

Fig. 14 illustrates a basic configuration of the shape analysis system 100. The shape analysis system 100 mainly includes a shape data acquisition device and the shape analysis device 50. The shape data acquisition device is, for example, the three-dimensional foot shape measuring device 18, or an information terminal 16 such as a mobile phone equipped with a camera function. A measurement subject 10 scans his or her foot shape using the three-dimensional foot shape measuring device 18. Alternatively, the measurement subject 10 may place his or her foot on a dedicated measurement mat 12 and scan his or her own foot shape by capturing an image of the foot with the camera function of the information terminal 16 and through measurement with a foot shape measurement application. For an opportunity to acquire foot shape data, for example, a person who is considering purchase of shoes at a shoe store or at home may be selected as a measurement subject 10.

The information terminal 16 may generate a foot shape of a measurement subject 10 by means of a three-dimensional scanner function using a technology such as light detection and ranging (LiDAR) or through image synthesis processing such as photogrammetry. The shape analysis system 100 may further include at least one of the measurement mat 12, the three-dimensional foot shape measuring device 18, or the information terminal 16. The information terminal 16 may be operated by the measurement subject 10 himself or herself as a user, or the three-dimensional foot shape measuring device 18 or the information terminal 16 may be used by a person other than the measurement subject 10, such as a salesperson of a shoe store, to perform the foot shape scanning. Therefore, the information terminal 16 may be a terminal owned by a shoe store where the three-dimensional foot shape measuring device 18 is installed or may be a terminal owned by the measurement subject 10 himself or herself.

The measurement values as the results of three-dimensional measurement of a foot shape scanned by the three-dimensional foot shape measuring device 18 or the information terminal 16 are transmitted from the three-dimensional foot shape measuring device 18 or the information terminal 16 to the shape analysis device 50. The shape analysis device 50 may be implemented as a server to which multiple three-dimensional foot shape measuring devices 18 or information terminals 16 connect via a network and that collects and analyzes measurement data from each of the three-dimensional foot shape measuring devices 18 or information terminals 16. The "body shape analysis device" in the claims may mean the entire shape analysis system 100 or may mean the shape analysis device 50. Also, in the present embodiment, the shape analysis device 50 substantially corresponds to the "body shape analysis device" because many of the characteristic functions included in the "body shape analysis device" in the claims are implemented such as to be included in the shape analysis device 50. The characteristic functions of the "body shape analysis device" may also be distributed between the information terminal 16 and the shape analysis device 50 or may also be implemented such that many of them are included in the information terminal 16.

The shape analysis device 50 is a server computer connected to multiple three-dimensional foot shape measuring devices 18 or multiple information terminals 16 via a network line, such as the Internet or a LAN, and a communication means, such as wireless communication. The information terminal 16 may be a mobile information terminal, such as a smartphone or a tablet terminal, or may be a personal computer.

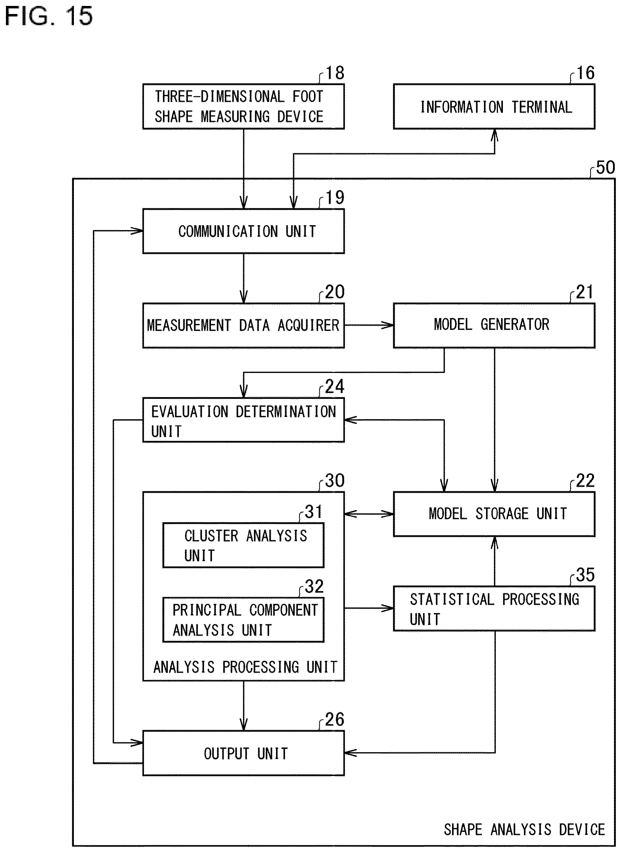

Fig. 15 is a functional block diagram that shows each configuration in the shape analysis device 50. The shape analysis device 50 may be implemented by various hardware configurations and software configurations. With regard to the shape analysis device 50 and the information terminal 16, Fig. 15 shows functional blocks implemented by coordination of various hardware configurations and software configurations. Therefore, it will be understood by those skilled in the art that these functional blocks may be implemented in a variety of forms by hardware only, software only, or a combination thereof. The information terminal 16 is constituted by a combination of hardware, such as a camera module, range sensor, microprocessor, touch panel, memory, and communication module. The information terminal 16 may be a general-purpose information terminal, such as a smartphone or tablet terminal, prepared by the measurement subject 10 or a shoe store. The information terminal 16 accesses a website provided by the shape analysis device 50 via a web browser or displays, on its screen, information provided from the shape analysis device 50, with application software running on the information terminal 16. The shape analysis device 50 of the present embodiment further includes, as its function, an evaluation determination unit 24, in addition to the functions of the shape analysis device 50 in the first embodiment. The communication unit 19 of the shape analysis device 50 is connected to the three-dimensional foot shape measuring device 18 and the information terminal 16 via a network.

Multiple foot shape three-dimensional homologous models to be stored in the model storage unit 22 may be classified into multiple clusters by the cluster analysis described in the first embodiment. Alternatively, the models may be three-dimensional homologous models that respectively correspond to last, footwear, or sock liner variations designed based on the classification results of the cluster analysis of the first embodiment. Alternatively, the models may be conventional three-dimensional homologous models that each correspond to a combination of a foot length and a ball girth in accordance with the JIS.

The measurement data acquirer 20 acquires, as measurement data, a three-dimensional coordinate group indicating anatomical features corresponding to a three-dimensional homologous model of the foot shape of the measurement subject 10, from the three-dimensional foot shape measuring device 18 or the information terminal 16 via the communication unit 19. The model generator 21 generates a foot shape three-dimensional homologous model based on the measurement data acquired by the measurement data acquirer 20 and outputs the model as a foot shape three-dimensional homologous model of the measurement subject 10 to the evaluation determination unit 24.

The evaluation determination unit 24 evaluates the similarity in coordinates between the three-dimensional coordinate group indicating anatomical features in the measurement data of the measurement subject 10 and the three-dimensional coordinate groups indicating anatomical features in multiple three-dimensional homologous models stored in the model storage unit 22. Based on the similarity in coordinates between corresponding three-dimensional homologous models, the evaluation determination unit 24 determines, among the multiple three-dimensional homologous models, a three-dimensional homologous model similar to the foot shape of the measurement subject 10.

More specifically, the evaluation determination unit 24 acquires a position vector from a predetermined reference point to each point in the multiple three-dimensional homologous models and also acquires a position vector from a predetermined reference point to each point in the three-dimensional homologous model in the measurement data. Based on the distance between points or the similarity between position vectors in corresponding three-dimensional homologous models, the evaluation determination unit 24 selects, from among the multiple three-dimensional homologous models, a three-dimensional homologous model similar to the foot shape of the measurement subject 10. The distance between points may be the Euclidean distance, and the similarity between position vectors may be the cosine similarity, for example. The evaluation determination unit 24 combines the selection result for a similar three-dimensional homologous model based on the Euclidean distance and the selection result for a similar three-dimensional homologous model based on the cosine similarity and determines therebetween the three-dimensional homologous model that is most similar to the foot shape of the measurement subject 10. It is assumed here that about 6,000 foot shape three-dimensional homologous models are stored in the model storage unit 22. In such a case, even if the evaluation determination unit 24 determines the most similar three-dimensional homologous model by calculating the similarity with all the three-dimensional homologous models, it can be determined in about 10 seconds of calculation, so that a foot shape can be extracted in a very short time.

The evaluation determination unit 24 scales the three-dimensional homologous model thus determined to various sizes based on the foot length of the measurement subject 10 and calculates the similarity between each of the scaled models and the foot shape three-dimensional homologous model of the measurement subject 10. Based on the similarity of each scaled model, the evaluation determination unit 24 determines the scale of the three-dimensional homologous model with the highest similarity. The evaluation determination unit 24 extracts, from the database in the model storage unit 22, the determined foot shape three-dimensional homologous model and the type of shoe or sock liner corresponding to the scale and generates information to be presented to the measurement subject 10.

The output unit 26 transmits, to the information terminal 16 via the communication unit 19, information on a foot shape model similar to a foot of the measurement subject 10 and recommendation information indicating the type of shoe or sock liner corresponding to the foot shape, determined by the evaluation determination unit 24.

In a modification, the evaluation determination unit 24 may evaluate which of the foot shape three-dimensional homologous models of multiple celebrities stored in the model storage unit 22 has the highest similarity, and the evaluation result may be output to the information terminal 16.

The present disclosure has been described based on embodiments. The embodiments are intended to be illustrative only, and it will be obvious to those skilled in the art that various modifications to a combination of constituting elements or processes in the embodiments could be developed and that such modifications also fall within the scope of the appended claims.

## Claims

1. A body shape analysis device (50), comprising:
a model storage unit (22) that is configured to store, in advance, a plurality of three-dimensional homologous models for a plurality of body shapes with a three-dimensional coordinate group indicating an anatomical feature of a foot, wherein a foot shape three-dimensional homologous model is a model with which multiple foot shapes having various sizes and shapes are each represented by a polyhedron anatomically defined by the same topological structure with the same number of points;
a measurement data acquirer (20) that is configured to acquire, as measurement data, a three-dimensional coordinate group indicating an anatomical feature corresponding to the three-dimensional homologous model of a body shape of a measurement subject; and
an evaluation determination unit (24) that is configured to determine, among the plurality of three-dimensional homologous models, a three-dimensional homologous model similar to a body shape of the measurement subject by evaluating similarity in coordinates between a three-dimensional coordinate group indicating an anatomical feature in the measurement data and a three-dimensional coordinate group indicating an anatomical feature in each of the plurality of three-dimensional homologous models,
**characterized in that**
the plurality of three-dimensional homologous models are classified into a plurality of clusters by cluster analysis based on cosine similarity between position vectors from a predetermined reference point to the respective points in the three-dimensional homologous models and are stored in the model storage unit.

2. The body shape analysis device (50) according to claim 1, wherein the evaluation determination unit (24) is configured:
to acquire a position vector from a predetermined reference point to each point in the plurality of three-dimensional homologous models,
to acquire a position vector from the predetermined reference point to each point in a three-dimensional homologous model in the measurement data, and
to determine, based on the distance between points or the similarity between position vectors in corresponding three-dimensional homologous models, a three-dimensional homologous model similar to a body shape of the measurement subject, among the plurality of three-dimensional homologous models.

3. The body shape analysis device (50) according to claim 1 or 2, wherein the plurality of three-dimensional homologous models are classified into a plurality of clusters in an upper layer by cluster analysis based on cosine similarity and are further classified into a plurality of more detailed clusters in a lower layer by performing cluster analysis on each cluster in the upper layer based on principal component analysis of which results are dimensionally reduced to the second and the subsequent principal components excluding the first principal component.

4. The body shape analysis device (50) according to any one of claims 1 through 3, wherein the model storage unit (22) is configured to further store a value indicating demand for each of a plurality of three-dimensional homologous models classified into a plurality of clusters, based on the number of the three-dimensional homologous models in each cluster for a plurality of clusters classified by cluster analysis.

5. A body shape analysis method, comprising:
reading a plurality of three-dimensional homologous models for a plurality of body shapes with a three-dimensional coordinate group indicating an anatomical feature of a foot, wherein a foot shape three-dimensional homologous model is a model with which multiple foot shapes having various sizes and shapes are each represented by a polyhedron anatomically defined by the same topological structure with the same number of points;
acquiring, as measurement data, a three-dimensional coordinate group indicating an anatomical feature corresponding to the three-dimensional homologous model of a body shape of a measurement subject;
evaluating similarity in coordinates between a three-dimensional coordinate group indicating an anatomical feature in the measurement data and a three-dimensional coordinate group indicating an anatomical feature in each of the plurality of three-dimensional homologous models; and
determining, among the plurality of three-dimensional homologous models, a three-dimensional homologous model similar to a body shape of the measurement subject, based on evaluation for the similarity,
**characterized in that**
the plurality of three-dimensional homologous models are classified into a plurality of clusters by cluster analysis based on cosine similarity between position vectors from a predetermined reference point to the respective points in the three-dimensional homologous models and are stored in the model storage unit.

## Patentansprüche

1. Körperformanalysevorrichtung (50), mit:
einer Modellspeichereinheit (22), die konfiguriert ist, um im Voraus eine Vielzahl von dreidimensionalen homologen Modellen für eine Vielzahl von Körperformen mit einer dreidimensionalen Koordinatengruppe speichert, die ein anatomisches Merkmal eines Fußes angibt, wobei ein dreidimensionales homologes Modell einer Fußform ein Modell ist, bei dem mehrere Fußformen mit verschiedenen Größen und Formen jeweils durch ein Polyeder dargestellt sind, das anatomisch durch dieselbe topologische Struktur mit derselben Anzahl von Punkten definiert ist;
einer Messdatenbezugseinrichtung (20), die konfiguriert ist, um als Messdaten eine dreidimensionale Koordinatengruppe zu beziehen, die ein anatomisches Merkmal angibt, das dem dreidimensionalen homologen Modell einer Körperform einer Messperson entspricht; und
einer Auswertungsbestimmungseinheit (24), die konfiguriert ist, um unter der Vielzahl von dreidimensionalen homologen Modellen ein dreidimensionales homologes Modell zu bestimmen, das einer Körperform des Messobjekts ähnelt, durch Auswerten einer Ähnlichkeit der Koordinaten zwischen einer dreidimensionalen Koordinatengruppe, die ein anatomisches Merkmal in den Messdaten angibt, und einer dreidimensionalen Koordinatengruppe, die ein anatomisches Merkmal in jedem der Vielzahl von dreidimensionalen homologen Modellen angibt,
**dadurch gekennzeichnet, dass**
die Vielzahl dreidimensionaler homologer Modelle durch Clusteranalyse auf der Grundlage der Kosinusähnlichkeit zwischen Positionsvektoren von einem vorbestimmten Referenzpunkt zu den jeweiligen Punkten in den dreidimensionalen homologen Modellen in eine Vielzahl von Clustern klassifiziert und in der Modellspeichereinheit gespeichert wird.

2. Körperformanalysevorrichtung (50) gemäß Anspruch 1**,** wobei die Auswertungsbestimmungseinheit (24) konfiguriert ist, um:
einen Positionsvektor von einem vorbestimmten Referenzpunkt zu jedem Punkt in der Vielzahl dreidimensionaler homologer Modelle zu beziehen,
einen Positionsvektor von dem vorbestimmten Referenzpunkt zu jedem Punkt in einem dreidimensionalen homologen Modell in den Messdaten zu beziehen, und
auf der Grundlage des Abstands zwischen Punkten oder der Ähnlichkeit zwischen Positionsvektoren in entsprechenden dreidimensionalen homologen Modellen ein dreidimensionales homologes Modell zu bestimmen, das einer Körperform des Messobjekts ähnelt, unter der Vielzahl von dreidimensionalen homologen Modellen.

3. Körperformanalysevorrichtung (50) gemäß Anspruch 1 oder 2, wobei die Vielzahl dreidimensionaler homologer Modelle in einer oberen Schicht durch Clusteranalyse auf der Grundlage der Kosinusähnlichkeit in eine Vielzahl von Clustern klassifiziert wird und durch Durchführung einer Clusteranalyse an jedem Cluster in der oberen Schicht auf der Grundlage einer Hauptkomponentenanalyse, deren Ergebnisse auf die zweite und die nachfolgenden Hauptkomponenten unter Ausschluss der ersten Hauptkomponente dimensional reduziert werden, in einer unteren Schicht weiter in eine Vielzahl detaillierterer Cluster klassifiziert wird.

4. Körperformanalysevorrichtung (50) gemäß einem der Ansprüche 1 bis 3, wobei die Modellspeichereinheit (22) konfiguriert ist, um weiterhin einen Wert zu speichern, der die Nachfrage für jedes einer Vielzahl von dreidimensionalen homologen Modellen angibt, die in eine Vielzahl von Clustern klassifiziert sind, basierend auf der Anzahl der dreidimensionalen homologen Modelle in jedem Cluster für eine Vielzahl von Clustern, die durch Clusteranalyse klassifiziert wurden.

5. Körperformanalyseverfahren, mit:
Einlesen einer Vielzahl von dreidimensionalen homologen Modellen für eine Vielzahl von Körperformen mit einer dreidimensionalen Koordinatengruppe, die ein anatomisches Merkmal eines Fußes angibt, wobei ein dreidimensionales homologes Fußformmodell ein Modell ist, bei dem mehrere Fußformen mit verschiedenen Größen und Formen jeweils durch einen Polyeder dargestellt werden, das anatomisch durch dieselbe topologische Struktur mit derselben Anzahl von Punkten definiert ist;
Beziehen einer dreidimensionalen Koordinatengruppe, die ein anatomisches Merkmal entsprechend dem dreidimensionalen homologen Modell einer Körperform eines Messobjekts angibt, als Messdaten;
Auswerten einer Ähnlichkeit der Koordinaten zwischen einer dreidimensionalen Koordinatengruppe, die ein anatomisches Merkmal in den Messdaten angibt, und einer dreidimensionalen Koordinatengruppe, die ein anatomisches Merkmal in jedem der mehreren dreidimensionalen homologen Modelle angibt; und
Bestimmen eines dreidimensionalen homologen Modells, das einer Körperform der Messperson ähnelt, aus der Vielzahl der dreidimensionalen homologen Modelle auf der Grundlage der Bewertung der Ähnlichkeit,
**dadurch gekennzeichnet, dass**
die Vielzahl dreidimensionaler homologer Modelle durch Clusteranalyse auf der Grundlage der Kosinusähnlichkeit zwischen Positionsvektoren von einem vorbestimmten Referenzpunkt zu den jeweiligen Punkten in den dreidimensionalen homologen Modellen in eine Vielzahl von Clustern klassifiziert und in der Modellspeichereinheit gespeichert wird.

## Revendications

1. Dispositif d'analyse de forme corporelle (50), comprenant :
une unité de stockage de modèle (22) qui est configurée pour stocker, d'avance, une pluralité de modèles homologues tridimensionnels pour une pluralité de formes corporelles avec un groupe de coordonnées tridimensionnelles indiquant une caractéristique anatomique d'un pied, dans lequel un modèle homologue tridimensionnel de forme de pied est un modèle avec lequel de multiples formes de pied ayant diverses tailles et formes sont chacune représentées par un polyèdre défini anatomiquement par la même structure topologique avec le même nombre de points ;
un organe d'acquisition de données de mesure (20) qui est configuré pour acquérir, en tant que données de mesure, un groupe de coordonnées tridimensionnelles indiquant une caractéristique anatomique correspondant au modèle homologue tridimensionnel d'une forme corporelle d'un sujet de mesure ; et
une unité de détermination d'évaluation (24) qui est configurée pour déterminer, parmi la pluralité de modèles homologues tridimensionnels, un modèle homologue tridimensionnel similaire à une forme corporelle du sujet de mesure en évaluant une similarité de coordonnées entre un groupe de coordonnées tridimensionnelles indiquant une caractéristique anatomique dans les données de mesure et un groupe de coordonnées tridimensionnelles indiquant une caractéristique anatomique dans chacun de la pluralité de modèles homologues tridimensionnels,
**caractérisé en ce que**
la pluralité de modèles homologues tridimensionnels sont classifiés en une pluralité de grappes, par analyse par grappes, sur la base d'une similarité cosinus entre des vecteurs de position allant d'un point de référence prédéterminé aux points respectifs dans les modèles homologues tridimensionnels, et sont stockés dans l'unité de stockage de modèle.

2. Dispositif d'analyse de forme corporelle (50) selon la revendication 1, dans lequel l'unité de détermination d'évaluation (24) est configurée :
pour acquérir un vecteur de position allant d'un point de référence prédéterminé à chaque point dans la pluralité de modèles homologues tridimensionnels,
pour acquérir un vecteur de position allant du point de référence prédéterminé à chaque point dans un modèle homologue tridimensionnel dans les données de mesure, et
pour déterminer, sur la base de la distance entre des points ou de la similarité entre des vecteurs de position dans des modèles homologues tridimensionnels correspondants, un modèle homologue tridimensionnel similaire à une forme corporelle du sujet de mesure, parmi la pluralité de modèles homologues tridimensionnels.

3. Dispositif d'analyse de forme corporelle (50) selon la revendication 1 ou 2, dans lequel la pluralité de modèles homologues tridimensionnels sont classifiés en une pluralité de grappes dans une couche supérieure, par analyse par grappes, sur la base d'une similarité cosinus, et sont en outre classifiés en une pluralité de grappes plus détaillées dans une couche inférieure en réalisant une analyse par grappes sur chaque grappe dans la couche supérieure, sur la base d'analyse en composantes principales dont des résultats sont réduits dimensionnellement à la deuxième composante principale et celle subséquente à l'exclusion de la première composante principale.

4. Dispositif d'analyse de forme corporelle (50) selon l'une quelconque des revendications 1 à 3, dans lequel l'unité de stockage de modèle (22) est configurée pour stocker en outre une valeur indiquant une demande en ce qui concerne chacun d'une pluralité de modèles homologues tridimensionnels classifiés en une pluralité de grappes, sur la base du nombre des modèles homologues tridimensionnels dans chaque grappe, pour une pluralité de grappes, classifiés par analyse par grappes.

5. Procédé d'analyse de forme corporelle, comprenant :
la lecture d'une pluralité de modèles homologues tridimensionnels pour une pluralité de formes corporelles avec un groupe de coordonnées tridimensionnelles indiquant une caractéristique anatomique d'un pied, dans lequel un modèle homologue tridimensionnel de forme de pied est un modèle avec lequel de multiples formes de pied ayant diverses tailles et formes sont chacune représentées par un polyèdre défini anatomiquement par la même structure topologique avec le même nombre de points ;
l'acquisition, en tant que données de mesure, d'un groupe de coordonnées tridimensionnelles indiquant une caractéristique anatomique correspondant au modèle homologue tridimensionnel d'une forme corporelle d'un sujet de mesure ;
l'évaluation d'une similarité de coordonnées entre un groupe de coordonnées tridimensionnelles indiquant une caractéristique anatomique dans les données de mesure et un groupe de coordonnées tridimensionnelles indiquant une caractéristique anatomique dans chacun de la pluralité de modèles homologues tridimensionnels ; et
la détermination, parmi la pluralité de modèles homologues tridimensionnels, d'un modèle homologue tridimensionnel similaire à une forme corporelle du sujet de mesure, sur la base d'une évaluation en ce qui concerne la similarité,
**caractérisé en ce que**
la pluralité de modèles homologues tridimensionnels sont classifiés en une pluralité de grappes, par analyse par grappes, sur la base d'une similarité cosinus entre des vecteurs de position allant d'un point de référence prédéterminé aux points respectifs dans les modèles homologues tridimensionnels, et sont stockés dans l'unité de stockage de modèle.
